(19) Europäisches Patentamt European Patent Office Office européen des brevets

(11) **EP 4 467 890 A1**

(12) **EUROPEAN PATENT APPLICATION**
published in accordance with Art. 153(4) EPC

(43) Date of publication:
**27.11.2024 Bulletin 2024/48**

(21) Application number: **23781170.8**

(22) Date of filing: **21.02.2023**

(51) International Patent Classification (IPC):
**F24F 11/62** (2018.01)     **F24F 11/89** (2018.01)
**F24F 11/50** (2018.01)     **F25B 49/02** (2006.01)
**G01N 33/00** (2006.01)     **F24F 110/10** (2018.01)
**F24F 110/20** (2018.01)     **F24F 110/30** (2018.01)
**F24F 110/64** (2018.01)

(52) Cooperative Patent Classification (CPC):
**F24F 11/89; F25B 49/02; G01N 33/00;**
F24F 2110/10; F24F 2110/20; F24F 2110/30;
F24F 2110/50; F24F 2110/62; F24F 2110/64

(86) International application number:
**PCT/KR2023/002423**

(87) International publication number:
**WO 2023/191311 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.03.2022 KR 20220038221**

(71) Applicant: **Samsung Electronics Co., Ltd
Suwon-si, Gyeonggi-do 16677 (KR)**

(72) Inventors:
• **HWANG, Jun**
  **Suwon-si Gyeonggi-do 16677 (KR)**
• **KIM, Seokkyun**
  **Suwon-si Gyeonggi-do 16677 (KR)**
• **KIM, Taewoo**
  **Suwon-si Gyeonggi-do 16677 (KR)**
• **SON, Kilsoo**
  **Suwon-si Gyeonggi-do 16677 (KR)**
• **LEE, Sangwoo**
  **Suwon-si Gyeonggi-do 16677 (KR)**
• **CHUNG, Ohkun**
  **Suwon-si Gyeonggi-do 16677 (KR)**

(74) Representative: **Gulde & Partner
Patent- und Rechtsanwaltskanzlei mbB
Wallstraße 58/59
10179 Berlin (DE)**

(54) **AIR CONDITIONING DEVICE, AIR SENSOR DEVICE, AND CONTROL METHOD THEREOF**

(57) Disclosed is an air conditioning device. The air conditioning device comprises: a communication interface including a circuit; a temperature sensor; a compressor; and a processor for controlling the operation of the compressor on the basis of a set temperature value set in the air conditioning device, a first temperature value acquired by the temperature sensor, and a second temperature value received from the air sensor device through the communication interface. The processor identifies the difference between the first temperature value and the second temperature value, and, when the identified difference is at least a threshold value, controls the operation of the compressor on the basis of the set temperature value and the first temperature value.

FIG. 1

EP 4 467 890 A1

**Description**

[Technical Field]

The disclosure relates to an air conditioning device, an air sensor device and a control method thereof, and more particularly to an air conditioning device that operates performing intercommunication, an air sensor device, and a control method thereof.

[Background Art]

Recently, air conditioning devices for maintaining a comfortable indoor environment by adjusting temperature, humidity, cleanliness, airflow, and the like of an indoor space are being supplied.

Because air conditioning devices of the related art operate taking into consideration a current indoor temperature and a setting temperature, there has been a disadvantage of not being able to operate taking into consideration a sensory temperature of a user, a surrounding temperature at which the user is positioned, or the like.

At times, there has been a disadvantage of causing displeasure to the user due to excessive cooling or excessive heating by continuously performing cooling or heating functions despite the sensory temperature of the user having reached a desired temperature of the user (or, the setting temperature of the user).

A portable air sensor device and the like may sense the surrounding temperature in which the user is positioned by being positioned adjacently to the user, and has an advantage of appropriately performing the cooling or heating function if the air conditioning device operates using a temperature value sensed by the air sensor device.

However, the above-described advantage is manifested only when the air sensor device is positioned adjacently to the user, or when positioned so as to appropriately sense the surrounding temperature at which the user is positioned, and has a problem of the disadvantage of causing displeasure to the user due to excessive cooling or excessive heating by continuously performing cooling or heating functions despite the sensory temperature of the user having reached the desired temperature of the user (or, the setting temperature of the user) still occurring if the air sensor device is not positioned adjacently to the user.

Accordingly, there has been a continuous demand for an air conditioning device that predicts (or, identifies) a position of the air sensor device, and appropriately uses the temperature value sensed by the air sensor device and a control method thereof.

[Disclosure]

[Technical Solution]

The disclosure addresses the above-described necessity, and an object of the disclosure is in providing an air conditioning device which operates in connection with an air sensor device, and operates by appropriately using a temperature value sensed by the air sensor device, the air sensor device, and a control method thereof.

According to an embodiment for achieving the above-described object of the disclosure, an air conditioning device includes a communication interface including circuitry, a temperature sensor, a compressor, and a processor configured to control an operation of the compressor based on a setting temperature value set from the air conditioning device, a first temperature value obtained by the temperature sensor, and a second temperature value received from an air sensor device through the communication interface, and the processor is configured to identify a difference value between the first temperature value and the second temperature value, and control, based on the identified difference value being greater than or equal to a threshold value, an operation of the compressor based on the setting temperature value and the first temperature value.

Here, the processor may be configured to identify, based on the identified difference value being greater than or equal to a threshold value, a relationship between a position at which air discharged from the air conditioning device reaches and a current position of the air sensor device, and transmit information that guides of a position change of the air sensor device based on the identified relationship to the air sensor device through the communication interface.

Here, the processor may be configured to identify, based on the identified difference value being greater than or equal to a threshold value, that the air sensor device is disposed at a position at which air discharged from the air conditioning device directly reaches, and transmit information that guides of the position change of the air sensor device to the air sensor device through the communication interface.

In addition, the processor may be configured to identify, based on the air conditioning device operating in a cooling mode, whether a difference value of subtracting the second temperature value from the first temperature value is greater than or equal to a first threshold value, and operate, based on the identified difference value being greater than or equal to the first threshold value, the compressor for a cooling function to be performed based on the setting temperature and the first temperature value.

Here, the processor may be configured to operate, based on the difference value being less than the first threshold value, the compressor for the cooling function to be performed based on the setting temperature and the second temperature value.

In addition, the processor may be configured to identify, based on the air conditioning device operating in a heating mode, whether a difference value of subtracting the first temperature value from the second temperature value is greater than or equal to a second threshold value, and operate, based on the identified difference value being greater than or equal to the second threshold value, the compressor for a heating function to be performed based on the setting temperature and the first temperature value.

In addition, the processor may be configured to operate, based on the difference value being less than the second threshold value, the compressor for the heating function to be performed based on the setting temperature and the second temperature value.

In addition, the processor may be configured to set a third threshold value as the threshold value based on the first temperature value and the second temperature value being obtained at a time-point at which a first time is passed from an operation starting time-point of the air conditioning device, and set a fourth threshold value different from the third threshold value as the threshold value based on the first temperature value and the second temperature value being obtained at a time-point at which a second time different from the first time is passed from an operation starting time-point of the air conditioning device.

Here, the processor may be configured to set the fourth threshold value to the third threshold value to a relatively small value based on the second time being relatively longer than the first time.

In addition, the processor may be configured to transmit the first temperature value to an external server through the communication interface, and control, based on a control signal for controlling an operation of the compressor being received from the external server according to the difference value of the first temperature value and the second temperature value, an operation of the compressor based on the control signal.

According to an embodiment for achieving the above-described object of the disclosure, a control method of an air conditioning device including a temperature sensor includes obtaining a first temperature value through the temperature sensor, and controlling an operation of a compressor of the air conditioning device based on a setting temperature value set from the air conditioning device, the first temperature value, and a second temperature value received from an air sensor device, and the controlling includes identifying a difference value between the first temperature value and the second temperature value, and controlling, based on the identified difference value being greater than or equal to a threshold value, an operation of the compressor based on the setting temperature value and the first temperature value.

Then, the control method may further include identifying, based on the identified difference value being greater than or equal to the threshold value, a relationship between a position at which air discharged from the air conditioning device reaches and a current position of the air sensor device, and transmitting information that guides of a position change of the air sensor device to the air sensor device based on the identified relationship.

Here, the identifying may include identifying, based on the identified difference value being greater than or equal to the threshold value, that the air sensor device is disposed at a position at which air discharged from the air conditioning device directly reaches.

In addition, the controlling may include identifying, based on the air conditioning device operating in a cooling mode, whether a difference value of subtracting the second temperature value from the first temperature value is greater than or equal to a first threshold value, and operating, based on the identified difference value being greater than or equal to the first threshold value, the compressor for a cooling function to be performed based on the setting temperature and the first temperature value.

Here, the controlling may include operating, based on the difference value being less than the first threshold value, the compressor for the cooling function to be performed based on the setting temperature and the second temperature value.

In addition, the controlling may include identifying, based on the air conditioning device operating in a heating mode, whether a difference value of subtracting the first temperature value from the second temperature value is greater than or equal to a second threshold value, and operating, based on the identified difference value being greater than or equal to the second threshold value, the compressor for a heating function to be performed based on the setting temperature and the first temperature value.

Here, the controlling may include operating, based on the difference value being less than the second threshold value, the compressor for the heating function to be performed based on the setting temperature and the second temperature value.

In addition, the control method may further include setting a third threshold value as the threshold value based on the first temperature value and the second temperature value being obtained at a time-point at which a first time is passed from an operation starting time-point of the air conditioning device, and setting a fourth threshold value different from the third threshold value as the threshold value based on the first temperature value and the second temperature value being obtained at a time-point at which a second time different from the first time is passed from an operation starting time-point of the air conditioning device.

Here, the setting a fourth threshold value may include setting the fourth threshold value to the third threshold value to a relatively small value based on the second time being relatively longer than the first time.

In addition, the control method may further include transmitting the first temperature value to an external server, and the controlling may include controlling, based on a control signal for controlling an operation of the compressor being received from the external server according to a difference value of the first temperature value and the second temperature value, an operation of the compressor based on the control signal.

According to various embodiments of the disclosure, based on the air sensor device being determined as appropriately sensing the surrounding temperature of the user, the air conditioning device may be operated using the temperature value sensed by the air sensor device, and based on the air sensor device being determined as not appropriately sensing the surrounding temperature of the user, the air conditioning device may be operated using the temperature value sensed by the air conditioning device.

Accordingly, the air conditioning device may operate taking into consideration a sensory temperature of the user, and an indoor temperature in which the user is positioned.

[Description of Drawings]

FIG. 1 is a diagram illustrating an air conditioning device and an air sensor device according to an embodiment of the disclosure;

FIG. 2 is a block diagram illustrating a configuration of an air conditioning device according to an embodiment of the disclosure;

FIG. 3 is a block diagram illustrating a configuration of an air sensor device according to an embodiment of the disclosure;

FIG. 4 is a diagram illustrating communication between an air conditioning device and an air sensor device according to an embodiment of the disclosure;

FIG. 5 is a diagram illustrating a difference between a first temperature value and a second temperature value according to an embodiment of the disclosure;

FIG. 6 is a diagram illustrating guide information according to an embodiment of the disclosure;

FIG. 7 is a diagram illustrating an incorrect position of an air sensor device according to another embodiment of the disclosure;

FIG. 8 is a diagram illustrating a heating mode according to another embodiment of the disclosure;

FIG. 9 is a flowchart illustrating a control method of an air conditioning device in a cooling mode according to an embodiment of the disclosure;

FIG 10 is a flowchart illustrating a control method of an air conditioning device in a heating mode according to an embodiment of the disclosure; and

FIG. 11 is a sequence diagram illustrating communication between an air conditioning device and an air sensor device according to an embodiment of the disclosure.

[Detailed Description of Exemplary Embodiments]

Terms used in the disclosure will be briefly described, and the disclosure will be described in detail.

The terms used in an embodiment of the disclosure are general terms selected that are currently widely used considering their function herein. However, the terms may change depending on intention, legal or technical interpretation, emergence of new technologies, and the like of those skilled in the related art. Further, in certain cases, there may be terms arbitrarily selected, and in this case, the meaning of the term will be disclosed in greater detail in the relevant description. Accordingly, the terms used herein are not to be understood simply as its designation but based on the meaning of the term and the overall context of the disclosure.

Various modifications may be made to the embodiments of the disclosure, and there may be various types of embodiments. Accordingly, specific embodiments will be illustrated in drawings, and described in detail in the detailed description. However, it should be noted that the various embodiments are not for limiting the scope of the disclosure to a specific embodiment, but they should be interpreted to include all modifications, equivalents or alternatives of the embodiments included in the ideas and the technical scopes disclosed herein. In case it is determined that in describing the embodiments, detailed description of related known technologies may unnecessarily confuse the gist of the disclosure, the detailed description will be omitted.

Terms such as "first," and "second" may be used in describing the various elements, but the elements are not to be limited by the terms. The terms may be used only to distinguish one element from another.

A singular expression includes a plural expression, unless otherwise clearly specified in context. It is to be understood that the terms such as "form" or "include" are used herein to designate a presence of a characteristic, number, step, operation, element, component, or a combination thereof, and not to preclude a presence or a possibility of adding one or more of other characteristics, numbers, steps, operations, elements, components or a combination thereof.

The term "module" or "part" used in the disclosure perform at least one function or operation, and may be implemented with a hardware or software, or implemented with a combination of hardware and software. In addition, a plurality of "modules" or a plurality of "parts," except for a "module" or a "part" which needs to be implemented as a specific hardware, may be integrated in at least one module and implemented as at least one processor (not shown).

An embodiment of the disclosure will be described in detail below with reference to the accompanied drawings to aid in the understanding of those of ordinary skill in the art. However, the disclosure may be implemented in various different forms and is not limited to the embodiments described herein. Further, in the drawings, parts not relevant to the description may be omitted, and like reference numerals may be used for like parts throughout the whole of the disclosure to clearly describe the disclosure.

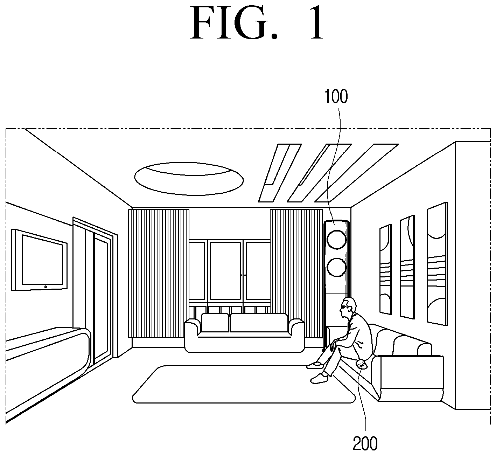

FIG. 1 is a diagram illustrating an air conditioning device and an air sensor device according to an embodiment of the disclosure.

An air conditioning device 100 according to an embodiment of the disclosure may refer to devices of various types which comfortably maintain indoors through heating, cooling, decreasing humidity (or, dehumidification), humidifying, ventilation, and the like as a conditioning device, an air adjusting device, or a conditioning system.

In an example, the air conditioning device 100 may be implemented as a hot air fan, a heater, an air-conditioner capable of both cooling and heating, and the like. However, the air conditioning device 100 is not limited to the above, and may be implemented in devices of various types which can increase and decrease indoor temperature, and the disclosure may be applied to even devices that can only operate any one from among cooling or heating. For convenience of description below, the air conditioning device 100 will be described as an air-conditioner capable of both cooling and heating.

The air conditioning device 100 according to an embodiment of the disclosure may include an indoor unit and an outdoor unit. The indoor unit may be connected with the outdoor unit, and the indoor unit may exchange refrigerant with the outdoor unit through a pipe. The air conditioning device 100 formed with the indoor unit and the outdoor unit may include various operation modes such as, cooling which lowers an indoor air temperature, heating which raises the indoor air temperature, wind blowing which forms an air flow indoors, dehumidification which lowers indoor humidity, and the like. The operation modes of the air conditioning device 100 will be described below.

The outdoor unit according to an embodiment of the disclosure may exchange heat with external air. The outdoor unit may exchange heat with external air through a cooling cycle that emits heat transferred through the refrigerant from the indoor unit to the outside, and exchanges heat with the external air through a heating cycle that absorbs heat lost by the refrigerant from the outside. The outdoor unit may include a compressor for compressing the refrigerant. Then, the compressor may be implemented into any one from among a constant speed type, a step type (or TPS), and an inverter type. The constant speed type may be a form that controls on and off of driving of the compressor according to a cooling and heating load. The step type may include a plurality of compressors, and may be a form that controls a number of compressors that drives according to the cooling and heating load. The inverter type may be a form that controls increasing and decreasing a driving capability of the compressor linearly according to the cooling and heating load.

The air conditioning device 100 according to an embodiment of the disclosure may perform communication with an air sensor device 200.

Here, the air sensor device 200 may mean a separate device distinguished from the air conditioning device 100 to monitor the temperature, air quality, and the like of indoor air.

For example, the air sensor device 200 may sense the temperature, humidity, fine dust concentration (e.g., PM 10, PM 2.5, and PM 1 values, etc.), and the like of surrounding air of the air sensor device 200.

The air sensor device 200 may be referred to as an air monitoring device, and the like, but will be collectively referred to as the air sensor device 200 for convenience of description below, and the air sensor device 200 shown in FIG. 1 is merely one example for convenience of description, and is not limited thereto. For example, the air sensor device 200 may also be implemented as a portable user terminal device that includes a sensor which can sense the temperature, air quality, and the like of the surrounding air.

The air sensor device 200 according to an embodiment of the disclosure may be implemented to be portable, and may sense a surrounding air temperature and a surrounding air quality of a user by being positioned adjacently to the user positioned indoors (e.g., an occupant (在室者)).

Specifically, the air sensor device 200 may transmit the sensed surrounding air temperature, surrounding air quality, and the like to the air conditioning device 100, and the air conditioning device 100 may perform an operation based on the surrounding air temperature and the surrounding air quality received from the air sensor device 200.

The air conditioning device 100 according to an embodiment of the disclosure may control an operation of the air conditioning device 100 by using the surrounding air temperature of the air sensor device 200 received from the air sensor device 200 rather than controlling an operation of the air conditioning device 100 (e.g., on and off of the compressor, etc.) using only the sensor (e.g., a temperature sensor) provided in the air conditioning device 100.

If the air sensor device 200 is positioned adjacently to the user, because the air conditioning device 100 operates using the surrounding air temperature of the user sensed through the air sensor device 200 in addition to the temperature sensed on its own, air of a more appropriate strength and temperature may be discharged in a surrounding environment of the user (e.g., the surrounding air temperature of the user).

In another example, if the air sensor device 200 is disposed somewhat spaced apart from the user, the surrounding air temperature sensed by the air sensor device 200 may be somewhat different from the surrounding air temperature of the user. In still another example, if the air sensor device 200 is disposed at a position at which air discharged from the air conditioning device 100 directly reaches, an air temperature sensed by the air sensor device 200 may be somewhat different from the surrounding air temperature of the user, or the indoor air temperature (hereinafter, an indoor temperature) in which the user is positioned.

In this case, if the air conditioning device 100 operates using the surrounding air temperature received from the air sensor device 200, a problem may occur of the air conditioning device 100 discharging air of an inappropriate strength and temperature (e.g., air of an excessive cooling or excessive heating temperature) in the surrounding environment of the user (e.g., the surrounding air temperature of the user).

According to various embodiments of the disclosure, a method for determining whether the air conditioning device 100 is disposed at a position at which the air sensor device 200 can appropriately sense the surrounding air temperature of the user using the surrounding air temperature received from the air sensor device 200 or a position at which the temperature of indoor air in which the user is positioned can be appropriately sensed will be described below.

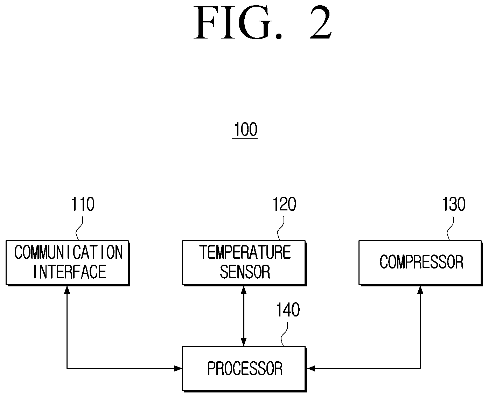

FIG. 2 is a block diagram illustrating a configuration of an air conditioning device according to an embodiment of the disclosure.

The air conditioning device 100 according to an embodiment may include a communication interface 110, a temperature sensor 120, a compressor 130, and a processor 140.

The communication interface 110 according to an embodiment of the disclosure may receive input of data of various types and information by performing communication with external devices. For example, the communication interface 110 may receive input of data of various types, information, and the like from home appliances (e.g., display device, air-conditioner, air purifier, etc.) external storage mediums (e.g., a USB memory), external servers (e.g., WEB HARD), and the like through communication methods such as, for example, and without limitation, an AP-based Wi-Fi (e.g., Wi-Fi, wireless LAN network), Bluetooth, ZigBee, a wired/wireless local area network (LAN), a wide area network (WAN), Ethernet, IEEE 1394, a high-definition multimedia interface (HDMI), a universal serial bus (USB), a mobile high-definition link (MHL), Audio Engineering Society/European Broadcasting Union (AES/EBU), Optical, Coaxial, or the like.

Specifically, the communication interface 110 according to an embodiment may perform communication with the air sensor device 200, and receive the temperature sensed by the air sensor device 200 from the air sensor device 200, for example, the surrounding air temperature of the air sensor device 200 (hereinafter, referred to as a second temperature value). However, the embodiment is not limited thereto, and may receive air quality sensed by the air sensor device 200 from the air sensor device 200, for example, humidity, fine dust concentration, and the like of the surrounding air of the air sensor device 200.

The air conditioning device 100 according to an embodiment of the disclosure may sense the temperature of the surrounding air (hereinafter, a first temperature value) of the air conditioning device 100 through the temperature sensor 120.

Meanwhile, the temperature of the surrounding air (i.e., the first temperature value) of the air conditioning device 100 sensed by the temperature sensor 120 may mean the indoor temperature in which the air conditioning device 100 is disposed. For example, the temperature sensor 120 may obtain the first temperature value by sensing the temperature of air (i.e., indoor air) suctioned through the indoor unit of the air conditioning device 100.

The air conditioning device 100 according to an embodiment of the disclosure may further include, in addition to the temperature sensor 120, a wind speed sensor which senses an indoor wind speed, a humidity sensor which senses the humidity in air, a fine dust sensor which senses the fine dust concentration, an olfactory sensor which senses smell, and the like, and may sense a surrounding air quality of the air conditioning device 100 using the sensors of various types.

The air conditioning device 100 according to an embodiment of the disclosure may include the compressor 130. Here, if the compressor 130 is operated according to control of the processor 140, the air conditioning device 100 may discharge heat transferred through the refrigerant to the outside when the air conditioning device 100 is in a cooling mode (i.e., decreasing indoor temperature), and the air conditioning device 100 may absorb heat lost by the refrigerant from the outside (i.e., increasing indoor temperature) when the air conditioning device 100 is in a heating mode.

According to an embodiment of the disclosure, the processor 140 may be implemented as a digital signal processor (DSP) that processes a digital signal, a microprocessor, or a time controller (TCON). However, the embodiment is not limited thereto, and may include, for example, and without limitation, one or more from among a central processing unit (CPU), a micro controller unit (MCU), a micro processing unit (MPU), a controller, an application processor (AP), a communication processor (CP), an ARM processor, or an artificial intelligence (AI) processor, or may be defined by the relevant term. In addition, the processor 140 may be implemented with a System on Chip (SoC) or a large scale integration (LSI) in which a processing algorithm is embedded, and may be implemented in a form of a field programmable gate array (FPGA). The processor 140 may perform various functions by executing computer executable instructions stored in a memory 130.

Specifically, the processor 140 may control an operation of the compressor 130 based on a setting temperature value set from the air conditioning device 100, the first temperature value obtained (or sensed) by the temperature sensor 120, and the second temperature value received from the air sensor device 200 through the communication interface 110.

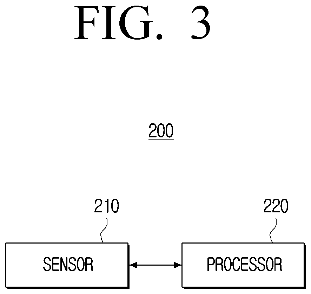

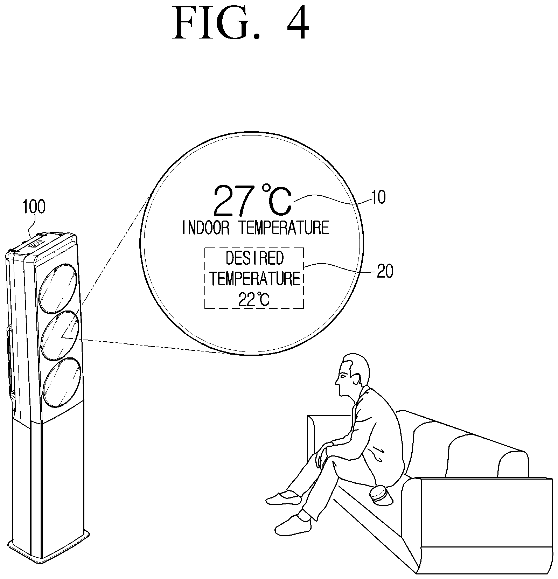

FIG. 3 is a block diagram illustrating a configuration of an air sensor device according to an embodiment of the disclosure. A detailed description thereof will be described below with reference to FIG. 4.

The air sensor device 200 according to an embodiment of the disclosure may include a sensor 210 and a processor 220.

The sensor 210 according to an embodiment may sense the surrounding air temperature (i.e., the second temperature value) of the air sensor device 200. In an example, if the air sensor device 200 is positioned adjacently to the user, the second temperature value sensed by the sensor 210 may correspond to the surrounding air temperature of the user.

Meanwhile, the sensor 210 may include a temperature sensor, and may further include, in addition to the temperature sensor, the wind speed sensor which senses the indoor wind speed, the humidity sensor which senses the humidity in air, the fine dust sensor which senses the fine dust concentration, the olfactory sensor which senses smell, and the like to sense the surrounding air quality of the air sensor device 200.

The processor 220 according to an embodiment of the disclosure may be implemented as the digital signal processor (DSP) that processes a digital signal, the microprocessor, or the time controller (TCON). However, the embodiment is not limited thereto, and may include, for example, and without limitation, one or more from among the central processing unit (CPU), the micro controller unit (MCU), the micro processing unit (MPU), the controller, the application processor (AP), the communication processor (CP), the ARM processor, or the artificial intelligence (AI) processor, or may be defined by the relevant term. In addition, the processor 220 may be implemented with the System on Chip (SoC) or the large scale integration (LSI) in which a processing algorithm is embedded, and may be implemented in the form of the field programmable gate array (FPGA). The processor 220 may perform various functions by executing computer executable instructions stored in the memory 130.

Specifically, the processor 220 may transmit the second temperature value sensed by the sensor 210, the surrounding air quality of the air sensor device 200, or the like to the air conditioning device 100 through a communication interface (not shown) provided in the air sensor device 200.

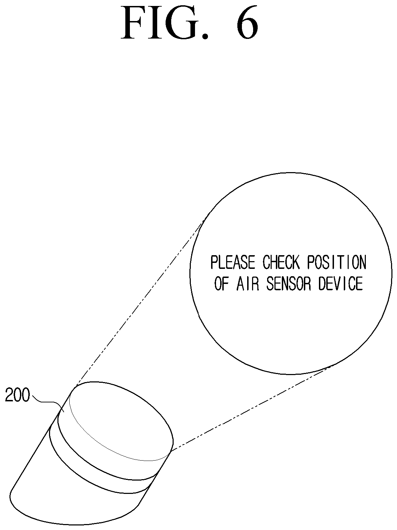

The processor 220 according to an embodiment may provide, based on a notification, guide information, or the like being received from the air conditioning device 100, provide the same. Detailed descriptions thereof will be described below with reference to FIG. 6.

FIG. 4 is a diagram illustrating communication between an air conditioning device and an air sensor device according to an embodiment of the disclosure.

Specifically, the processor 140 may control an operation of the compressor 130 based on a setting temperature value 20 set from the air conditioning device 100, a first temperature value 10 obtained (or, sensed) by the temperature sensor 120, the second temperature value received from the air sensor device 200 through the communication interface 110.

Here, the setting temperature value 20 may mean an indoor temperature to be reached by operating the air conditioning device 100, and may be referred to as a desired temperature. For example, the air conditioning device 100 may operate the compressor 130 until the first temperature value 10 obtained by the temperature sensor 120 reaches the setting temperature value 20 corresponding to a user setting.

Meanwhile, according to an embodiment of the disclosure, the air conditioning device 100 may operate the compressor 130 until the second temperature value obtained by the air sensor device 200 reaches the setting temperature value 20 in addition to the first temperature value 10 obtained by the temperature sensor 120.

For example, the air sensor device 200 may be positioned adjacently to the user, and in this case, the second temperature value obtained by the air sensor device 200 may correspond to the surrounding air temperature of the user. Accordingly, the air conditioning device 100 may control an operation of the compressor 130 (i.e., may adjust strength and temperature of air discharged by the air conditioning device 100) for the surrounding air temperature of the user to reach the setting temperature value 20 (or, until the surrounding air temperature of the user reaches the setting temperature value 20).

In another example, if the air sensor device 200 is not positioned adjacently to the user, the second temperature value obtained by the air sensor device 200 may be different from the surrounding air temperature of the user. In still another example, if the air sensor device 200 is disposed at a position at which air discharged from the air conditioning device 100 directly reaches, the second temperature value obtained by the air sensor device 200 may be different from the indoor temperature. Even in this case, if the air conditioning device 100 operates using the surrounding air temperature received from the air sensor device 200, a problem of the air conditioning device 100 discharging air of an inappropriate strength and temperature (e.g., air of excessive cooling or excessive heating) to the surrounding environment of the user (e.g., the surrounding air temperature of the user) may occur.

According to an embodiment, a method in which the air conditioning device 100 determines, by using the second temperature value received from the air sensor device 200, whether the air sensor device 200 is disposed at a position at which the surrounding air temperature of the user can be appropriately sensed or, at a position at which the indoor air temperature in which the user is positioned can be appropriately sensed will be described.

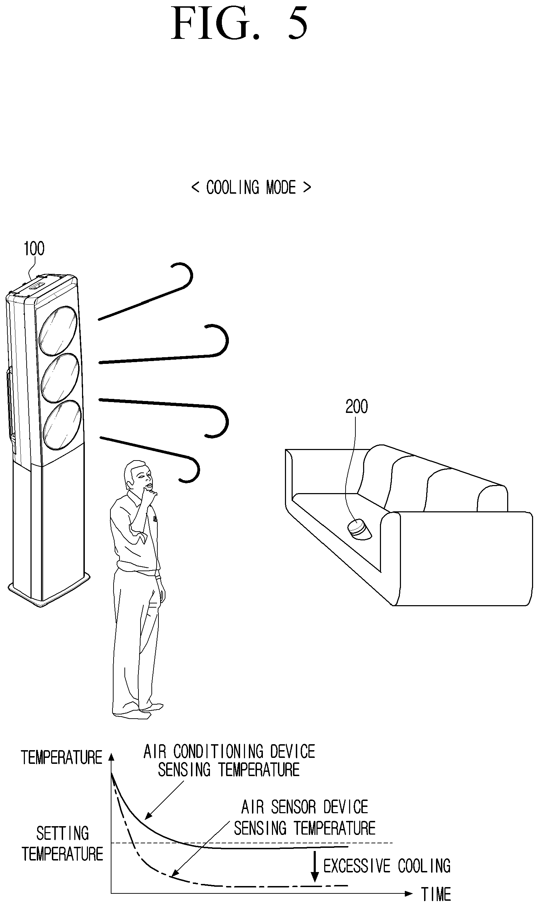

FIG. 5 is a diagram illustrating a difference between a first temperature value and a second temperature value according to an embodiment of the disclosure.

Referring to FIG. 5, the air conditioning device 100 according to an embodiment of the disclosure may operate in the cooling mode.

The processor 140 according to an embodiment may identify, based on the second temperature value being received from the air sensor device 200, a difference value between the first temperature value 10 (i.e., the temperature value obtained by the temperature sensor 120 provided in the air conditioning device 100) and the second temperature value.

Then, the processor 140 may control, based on the difference value being greater than or equal to a threshold value, an operation of the compressor 130 based on the first temperature value 10 and the setting temperature value 20.

The processor 140 according to an embodiment may identify, based on the difference between the first temperature value 10 and the second temperature value being greater than or equal to a threshold value, a relationship between the position at which air discharged from the air conditioning device 100 reaches and a current position of the air sensor device 200.

For example, if the air conditioning device 100 operates in the cooling mode, it may be assumed that the air sensor device 200 is disposed at a position at which air discharged from the air conditioning device 100 directly reaches.

In this case, the second temperature value obtained by the air sensor device 200 which is disposed at a position at which air discharged from the air conditioning device 100 directly reaches may differ from the first temperature value 10 obtained by the air conditioning device 100 by a threshold value or more prior to the air discharged from the air conditioning device 100 being diffused indoors, or the indoor temperature decreasing.

For example, a time-point prior to the air discharged from the air conditioning device 100 being diffused indoors, or prior to the indoor temperature decreasing may mean a time-point at which a first time is passed from an operation starting time-point of the air conditioning device 100.

The processor 140 according to an embodiment may identify a difference value between the first temperature value 10 and the second temperature value at a time-point at which the first time is passed from the operation starting time-point of the air conditioning device 100. Here, the first time may be between 3 minutes to 5 minutes, but this is merely one example, and may be variously changed.

Then, the processor 140 may identify, based on the difference value between the first temperature value 10 and the second temperature value being greater than or equal to a threshold value, that the air sensor device 200 is disposed at a position at which air discharged from the air conditioning device 100 directly reaches.

[Equation 1]

$$\text{Tr}_{\text{air-conditioningdevice}} - \text{Tr}_{\text{airsensordevice}} \geq 5°C$$

Here, the $\text{Tr}_{\text{air-conditioningdevice}}$ may be the first temperature value, $\text{Tr}_{\text{airsensordevice}}$ may be the second temperature value, and 5°C may be an example of the threshold value when in the cooling mode.

The processor 140 may identify, based on Equation 1 being satisfied, that air sensor device 200 is disposed at a position at which air discharged from the air conditioning device 100 directly reaches, and that the air sensor device 200 is not disposed at a position at which the surrounding air temperature of the user can be appropriately sensed or, at a position at which the indoor air temperature in which the user is positioned can be appropriately sensed (i.e., identify as the air sensor device 200 being disposed at an incorrect position). Then, the processor 140 may operate the compressor 130 using the first temperature value 10 and the setting temperature value 20.

As shown in FIG. 5, if the air conditioning device 100 is in the cooling mode, the air conditioning device 100 may discharge relatively cool air. According to an embodiment, if the air sensor device 200 is disposed at a position at which the cool air directly reaches, the second temperature value obtained by the air sensor device 200 may be relatively lower than the first temperature value 10 obtained by the air conditioning device 100.

In this case, according to a method of the related art, there is concern that the processor 140 may turn-off, based on determining that the indoor temperature has reached the setting temperature value 20 based on the second temperature value received from the air sensor device 200, the compressor 130 by erroneously determination despite the indoor temperature not having reached the setting temperature value 20. Accordingly, the processor 140 according to an embodiment of the disclosure may operate, based on the difference value between the first temperature value 10 and the second temperature value being greater than or equal to a threshold value, the compressor 130 for a cooling function to be performed using the first temperature value 10 and the setting temperature value 20.

In another example, the processor 140 may identify, based on the difference value between the first temperature value 10 and the second temperature value being less than a threshold value, that the air sensor device 200 is disposed at a position at which the surrounding air temperature of the user can be appropriately sensed or, at a positon the indoor air temperature in which the user is positioned can be appropriately sensed. Then, the processor 140 may operate the compressor 130 for the cooling function to be performed using the second temperature value and the setting temperature value 20.

For example, the processor 140 may maintain an operation of the compressor 130 based on the difference value between the first temperature value 10 and the second temperature value being less than a threshold value, and the second temperature value not having reached the setting temperature value 20. In another example, the processor 140 may stop the operation of the compressor 130 based on the second temperature value reaching the setting temperature value 20.

The processor 140 according to an embodiment of the disclosure may identify, based on the difference value between the first temperature value 10 and the second temperature value being greater than or equal to a threshold value (e.g., the first temperature value 10 being greater than the second temperature value by the threshold value or more), that the air sensor device 200 is disposed at a position at which air discharged from the air conditioning device 100 directly reaches, and transmit information guiding of a position change of the air sensor device 200 to the air sensor device 200 through the communication interface 110.

FIG. 6 is a diagram illustrating guide information according to an embodiment of the disclosure.

Referring to FIG. 6, the air sensor device 200 may display information guiding of the position change.

For example, the processor 140 of the air conditioning device 100 may identify, based on the difference value between the first temperature value 10 and the second temperature value being greater than or equal to a threshold value, that the air sensor device 200 is disposed at a position at which air discharged from the air conditioning device 100 directly reaches.

Then, the air conditioning device 100 may transmit guide information which shows a position change of the air sensor device 200 being required (e.g., 'please check the position of the air sensor device') to the air sensor device 200 for the air sensor device 200 to be disposed at a position at which the surrounding air temperature of the user can be appropriately sensed or, at a position at which the indoor air temperature in which the user is positioned can be appropriately sensed.

Then, the air sensor device 200 may display the guide information. In another example, the air sensor device 200 may also output a sound corresponding to the guide information (e.g., a sound corresponding to 'please check the position of the air sensor device') through a speaker (not shown).

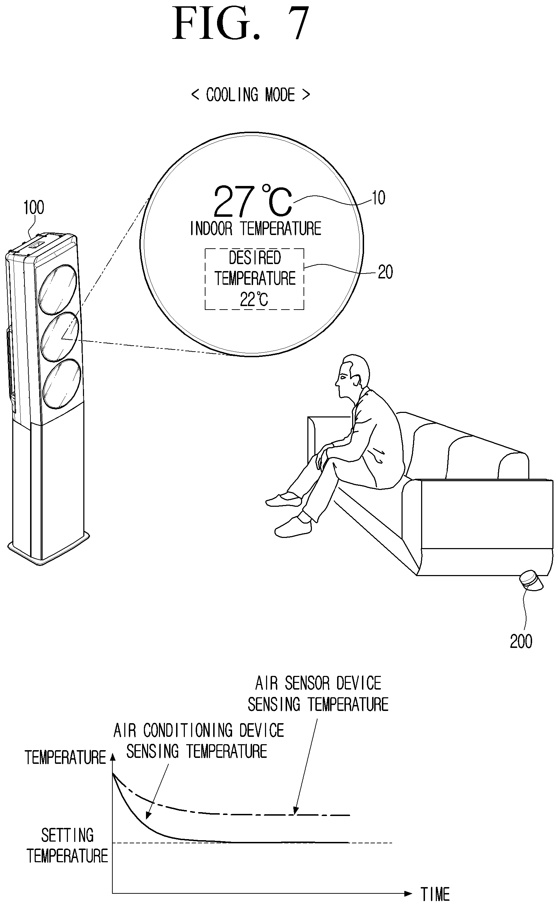

FIG. 7 is a diagram illustrating an incorrect position of an air sensor device according to another embodiment of the disclosure.

FIG. 7 shows, according to another embodiment of the disclosure, the air sensor device 200 not being disposed at a position at which the indoor air temperature in which the user is positioned can be appropriately sensed such as, in addition to a position at which air discharged from the air conditioning device 100 directly reaches, the air sensor device being disposed indoors different from the air conditioning device 100, or under furniture disposed at another indoors.

The processor 140 according to an embodiment may identify, based on the second temperature value being received from the air sensor device 200, the difference value between the first temperature value 10 (i.e., the temperature value obtained by the temperature sensor 120 provided in the air conditioning device 100) and the second temperature value.

According to an embodiment, the second temperature value obtained by the air sensor device 200 which is disposed at a position at which air discharged from the air conditioning device 100 is not able to reach (e.g., indoors different from the air conditioning device 100) may differ from the first temperature value 10 obtained by the air conditioning device 100 by a threshold value or more even after the air discharged from the air conditioning device 100 has been sufficiently diffused or the indoor temperature has decreased.

As shown in FIG. 7, if the air conditioning device 100 is in the cooling mode, the air conditioning device 100 may discharge cool air. According to an embodiment, if the air sensor device 200 is disposed at a position at which air discharged from the air conditioning device 100 it not able to reach, the second temperature value obtained by the air sensor device 200 may be relatively higher than the first temperature value 10 obtained by the air conditioning device 100 (e.g., a temperature value higher than the first temperature value 10 by a threshold value or more).

In this case, according to a method of the related art, there is concern that the processor 140 may continuously turn-on, based on determining that the indoor temperature has not reached the setting temperature value 20 based on the second temperature value received from the air sensor device 200, the compressor 130 by erroneously determination despite the indoor temperature having reached the setting temperature value 20. Accordingly, the processor 140 according to an embodiment of the disclosure may turn-off, based on the difference value between the first temperature value 10 and the second temperature value being greater than or equal to the threshold value (e.g., the second temperature value being greater than the first temperature value 10 by the threshold value or more), the compressor 130 using the first temperature value 10 and the setting temperature value 20.

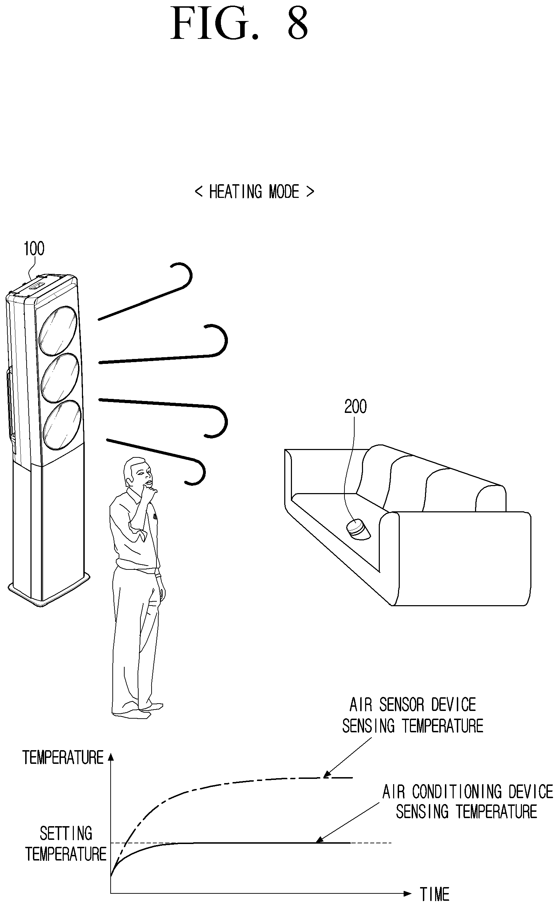

FIG. 8 is a diagram illustrating a heating mode according to another embodiment of the disclosure.

According to an embodiment, it may be assumed that the air conditioning device 100 is operating in the heating mode, and that the air sensor device 200 is disposed at a position at which air discharged from the air conditioning device 100 directly reaches.

In this case, the second temperature value obtained by the air sensor device 200 which is disposed at a position at which air discharged from the air conditioning device 100 directly reaches may differ from the first temperature value 10 obtained by the air conditioning device 100 by a threshold value or more prior to the air discharged from the air conditioning device 100 being diffused indoors, or the indoor temperature decreasing.

The processor 140 according to an embodiment of the disclosure may identify, based on the difference value between the first temperature value 10 and the second temperature value being greater than or equal to the threshold value, that the air sensor device 200 is disposed at a position at which air discharged from the air conditioning device 100 directly reaches.

$$[Equation\ 2]$$

$$\mathrm{Tr_{airsensordevice}} - \mathrm{Tr_{air\text{-}conditioningdevice}} \geq 7°C$$

Here, the $\mathrm{Tr_{air\text{-}conditioningdevice}}$ may be the first temperature value, the $\mathrm{Tr_{airsensordevice}}$ may be the second temperature value, and 7°C is an example of the threshold value when in the heating mode. The threshold value for when in the cooling mode and the threshold value for when in the heating mode may vary.

The processor 140 may identify, based on Equation 2 being satisfied, that air sensor device 200 is disposed at a position at which air discharged from the air conditioning device 100 directly reaches, and that the air sensor device 200 is not disposed at a position at which the surrounding air temperature of the user can be appropriately sensed or, at a position at which the indoor air temperature in which the user is positioned can be appropriately sensed (i.e., identify as the air sensor device 200 being disposed at an incorrect position). Then, the processor 140 may operate the compressor 130 using the first temperature value 10 and the setting temperature value 20.

As shown in FIG. 8, if the air conditioning device 100 is in the heating mode, the air conditioning device 100 may discharge relatively warm air. According to an embodiment, if the air sensor device 200 is disposed at a position at which the warm air directly reaches, the second temperature value obtained by the air sensor device 200 may be relatively higher than the first temperature value 10 obtained by the air conditioning device 100.

In this case, according to a method of the related art, there is concern that the processor 140 may turn-off, based on determining that the indoor temperature has reached the setting temperature value 20 based on the second temperature value received from the air sensor device 200, the compressor 130 by erroneously determination despite the indoor temperature not having reached the setting temperature value 20. Accordingly, the processor 140 according to an embodiment of the disclosure may operate, based on the difference value between the first temperature value 10 and the second temperature value being greater than or equal to the threshold value, the compressor 130 for a heating function to be performed using the first temperature value 10 and the setting temperature value 20.

In another example, the processor 140 may identify, based on the difference value between the first temperature value 10 and the second temperature value being less than the threshold value, that the air sensor device 200 is disposed at a position at which the surrounding air temperature of the user can be appropriately sensed or, at a position at which the indoor air temperature in which the user is positioned can be appropriately sensed. Then, the processor 140 may operate the compressor 130 for the heating function to be performed using the second temperature value and the setting temperature value 20.

For example, the processor 140 may maintain, based on the difference value between the first temperature value 10 and the second temperature value being less than the threshold value, the operation of the compressor 130 if the second temperature value has not reached the setting temperature value 20. In another example, the processor 140 may stop, based on the second temperature value having reached the setting temperature 20, an operation of the compressor 130.

The processor 140 according to an embodiment of the disclosure may identify, based on the difference value between the first temperature value 10 and the second temperature value being greater than or equal to the threshold value (e.g., the second temperature value being greater than the first temperature value 10 by the threshold value or more), that the air sensor device 200 is disposed at a position at which air discharged from the air conditioning device 100 directly reaches, and transmit information guiding of the position change of the air sensor device 200 to the air sensor device 200 through the communication interface 110.

In still another example, if the air conditioning device 100 operates in the heating mode as in the embodiment shown in FIG. 7, the processor 140 may determine, based on the difference value between the first temperature value 10 and the second temperature value being greater than or equal to the threshold value (e.g., the first temperature value 10 being greater than the second temperature value by the threshold value or more), that the air sensor device 200 is not disposed at a position at which the indoor air temperature in which the user is positioned can be appropriately sensed such as an indoors different from the air conditioning device 100, or under a furniture disposed in another indoors, or outdoors. Then, the processor 140 may operate the compressor 130 for the heating function to be performed using the first temperature value 10 and the setting temperature value 20.

Referring back to FIG. 5, the processor 140 according to an embodiment of the disclosure may set, based on the first temperature value 10 and the second temperature value being obtained at a time-point at which the first time (e.g., 3 minutes to 5 minutes) is passed from the operation starting time-point of the air conditioning device 100, a first threshold value as the threshold value.

In addition, the processor 140 may set, based on the first temperature value 10 and the second temperature value being obtained at a time-point at which the second time (e.g., 10 minutes) is passed from the operation starting time-point of the air conditioning device 100, a second threshold value as the threshold value.

For example, if the air conditioning device 100 operates in the cooling mode, the processor 140 may identify, in the first time, whether the difference value between the first temperature value 10 and the second temperature value is greater than or equal to the first threshold value (e.g., 5°C) based on Equation 1.

In another example, if the air conditioning device 100 operates in the cooling mode, the processor 140 may identify, in the second time, whether the difference value between the first temperature value 10 and the second temperature value is greater than or equal to the second threshold value (e.g., 3°C) based on Equation 3.

[Equation 3]

$$\text{Tr}_{\text{airsensordevice}} - \text{Tr}_{\text{air-conditioningdevice}} \geq 3°C$$

Here, the $\text{Tr}_{\text{air-conditioningdevice}}$ may be the first temperature value, the $\text{Tr}_{\text{airsensordevice}}$ may be the second temperature value, and 3°C may be an example of the second threshold value when in the cooling mode.

According to an embodiment, the threshold value corresponding to the second time (i.e., the second threshold value) may be a value relatively lower than the threshold value corresponding to the first time (i.e., the first threshold value).

Referring back to FIG. 8, in another example, if the air conditioning device 100 operates in the heating mode, the processor 140 may identify, in the first time, whether the difference value between the first temperature value 10 and the second temperature value is greater than or equal to the first threshold value (e.g., 7°C) based on Equation 2.

In another example, if the air conditioning device 100 operates in the heating mode, the processor 140 may identify, in the second time, whether the difference value between the first temperature value 10 and the second temperature value is greater than or equal to the second threshold value (e.g., 7°C) based on Equation 4.

[Equation 4]

$$\text{Tr}_{\text{air-conditioningdevice}} - \text{Tr}_{\text{airsensordevice}} \geq 5°C$$

Here, the $\text{Tr}_{\text{air-conditioningdevice}}$ may be the first temperature value, the $\text{Tr}_{\text{airsensordevice}}$ may be the second temperature value, and 5°C may be an example of the second threshold value when in the heating mode.

According to an embodiment, the threshold value corresponding to the second time (i.e., the second threshold value) may be a value relatively smaller than the threshold value corresponding to the first time (i.e., the first threshold value).

Meanwhile, the first time and the second time may be variously changed according to a manufacturer of the air conditioning device 100, a user setting, time at which the air sensor device 200 transmits the second temperature value, or the like. In another example, the first time and the second time may be changed according to the setting temperature value 20, the first temperature value 10, the second temperature value, and the like. For example, if a difference between the setting temperature value 20 and the first temperature value 10 is relatively great from the operation starting time-point of the air conditioning device 100, the processor 140 may set the first time to a time longer than 3 minutes to 5 minutes (e.g., 7 minutes), and set the second time to after a certain time (e.g., 5 minutes) from the first time. However, this is merely one example, and is not limited thereto.

The various embodiments of the disclosure may be performed by the processor 140 provided in the air conditioning device 100, but is not limited thereto, and may be performed by the processor 220 provided in the air sensor device 200.

For example, the air sensor device 200 may transmit a signal controlling an operation of the compressor to the air conditioning device 100 after identifying whether the difference between the first temperature value 10 and the second temperature value is greater than or equal to the threshold value,

In still another example, the air conditioning device 100 may transmit the first temperature value 10 to an external server, and the air sensor device 200 may transmit the second temperature value to the external server. Then, the external server may transmit, after identifying whether the difference between the first temperature value and the second temperature value is greater than or equal to the threshold value, a signal controlling an operation of the compressor to the air conditioning device 100.

According to various embodiments of the disclosure, the air conditioning device 100 may identify the difference value between the first temperature value 10 and the second temperature value, and identify whether the identified difference value is greater than or equal to the threshold value. Then, the air conditioning device 100 may control an operation of the air conditioning device 100 using the first temperature value 10 without taking into consideration the second temperature value received from the air sensor device 200 according to the identified result. However, the above is merely one example, and is not limited thereto.

For example, the air conditioning device 100 may identify a difference value between a first humidity (e.g., humidity sensed by a sensor provided in the air conditioning device 100) and a second humidity (e.g., humidity received from the air sensor device 200), and identify whether the identified difference value is greater than or equal to a threshold value. Then, the air conditioning device 100 may control an operation of the air conditioning device 100 using the first humidity without taking into consideration the second humidity received from the air sensor device 200 according to the identified result.

In another example, the air conditioning device 100 may identify a difference value between a first amount of fine dust (e.g., an amount of fine dust sensed by a sensor provided in the air conditioning device 100) and a second amount of fine dust (e.g., an amount of fine dust received from the air sensor device 200), and identify whether the identified difference value is greater than or equal to a threshold value. Then, the air conditioning device 100 may control an operation of the air conditioning device 100 using the first amount of fine dust without taking into consideration the second amount of fine dust received from the air sensor device 200 according to the identified result.

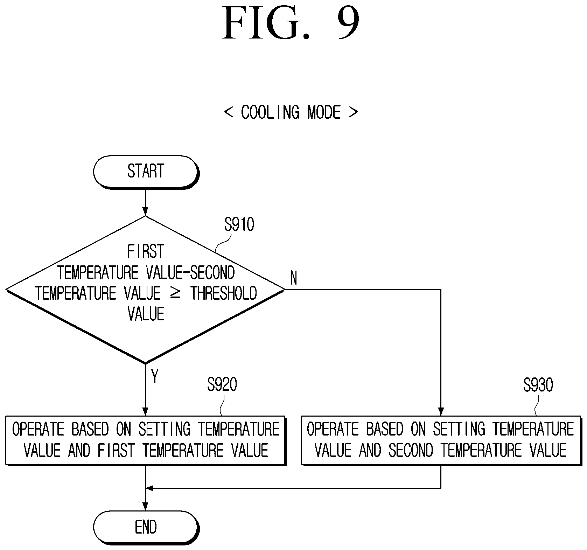

FIG. 9 is a flowchart illustrating a control method of an air conditioning device in a cooling mode according to an embodiment of the disclosure.

A control method of the air conditioning device according to an embodiment for achieving the above-described object of the disclosure may include, first, obtaining the first temperature value through the temperature sensor.

Then, an operation of the compressor of the air conditioning device may be controlled based on the setting temperature value set from the air conditioning device, the first temperature value, and the second temperature value received from the air sensor device.

The controlling an operation of the compressor according to an embodiment may include identifying, based on the air conditioning device operating in the cooling mode, a difference value of subtracting the second temperature value from the first temperature value (S910), and controlling, based on the identified difference value being greater than or equal to a threshold value (S910: Y), an operation of the compressor based on the setting temperature value and the first temperature value (S920).

According to an embodiment, identifying, based on the identified difference value being greater than or equal to a threshold value (S910: Y), a relationship between a position at which air discharged from the air conditioning device reaches and a current position of the air sensor device, and transmitting information guiding of the position change of the air sensor device to the air sensor device based on the identified relationship may be further included.

Step S910 of identifying according to an embodiment of the disclosure may include identifying, based on the identified difference value being greater than or equal to a threshold value (S910: Y), that the air sensor device is disposed at a position at which air discharged from the air conditioning device directly reaches.

According to another embodiment of the disclosure, operating, based on the difference value being less than a threshold value (S910: N), the compressor for the cooling function to be performed based on the setting temperature and the second temperature value may be included (S930).

In FIG. 9, for convenience of description, the controlling an operation of the compressor has been described as identifying whether the difference value of subtracting the second temperature value from the first temperature value is greater than or equal to the threshold value, but the above is not limited thereto.

For example, the controlling an operation of the compressor may include identifying the difference value between the first temperature value and the second temperature value, and controlling, based on the identified difference value being greater than or equal to the threshold value (e.g., identified difference value ≤ (-)threshold value or, (+)threshold value ≤ identified difference value), an operation of the compressor based on the setting temperature value and the first temperature value.

In another example, the controlling an operation of the compressor may include controlling, based on the identified difference value being less than the threshold value (e.g., (-)threshold value ≤ identified difference value ≤ (+) threshold value), an operation of the compressor based on the setting temperature value and the first temperature value.

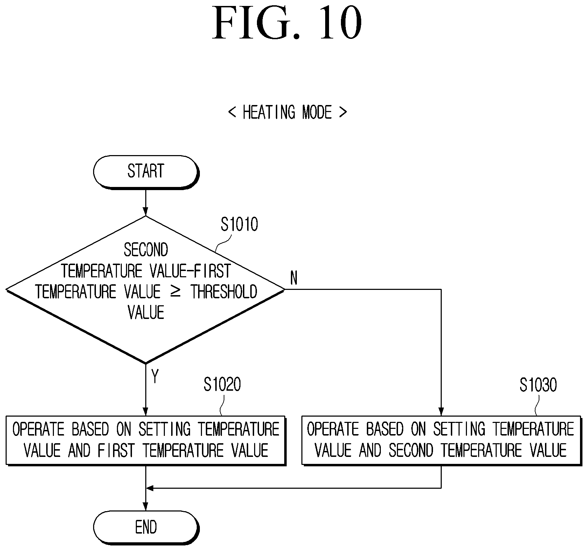

FIG 10 is a flowchart illustrating a control method of an air conditioning device in a heating mode according to an embodiment of the disclosure.

The controlling an operation of the compressor according to an embodiment of the disclosure may include identifying, based on the air conditioning device operating in the heating mode, a difference value of subtracting the first temperature value from the second temperature value (S1010), and controlling, based on the identified difference value being greater than or equal to the threshold value (S1010: Y), an operation of the compressor based on the setting temperature value and the first temperature value (S1020).

According to an embodiment, based on the identified difference value being greater than or equal to the threshold value (S1010: Y), identifying a relationship between a position at which air discharged from the air conditioning device reaches and a current position of the air sensor device and transmitting information guiding of the position change of the air sensor device to the air sensor device based on the identified relationship may be further included.

Step 1010 of identifying according to an embodiment of the disclosure may include identifying, based on the identified difference value being greater than or equal to the threshold value (S1010: Y), that the air sensor device is disposed at a position at which air discharged from the air conditioning device directly reaches.

According to another embodiment of the disclosure, based on the difference value being less than the threshold value (S1010: N), operating the compressor for the cooling function to be performed based on the setting temperature and the second temperature value (S1030) may be included.

In FIG. 10, for convenience of description, the controlling an operation of the compressor has been described as identifying whether the difference value of subtracting the first temperature value from the second temperature value is greater than or equal to the threshold value, but the above is one example and is not limited thereto.

For example, the controlling an operation of the compressor may include identifying the difference value between the first temperature value and the second temperature value, and controlling, based on the identified difference value being greater than or equal to the threshold value (e.g., identified difference value ≤ (-)threshold value or, (+)threshold value ≤ identified difference value), an operation of the compressor based on the setting temperature value and the first temperature value.

In another example, the controlling an operation of the compressor may include controlling, based on the identified difference value being less than the threshold value (e.g., (-)threshold value ≤ identified difference value ≤ (+) threshold value), an operation of the compressor based on the setting temperature value and the first temperature value. Meanwhile, according to an embodiment, the threshold value when in the cooling mode and the threshold value when in the heating mode may be the same, and may be different. For example, the threshold value when in the cooling mode may be a value relatively smaller than the threshold value when in the heating mode.

The control method according to an embodiment of the disclosure may further include setting a third threshold value as a threshold value based on the first temperature value and the second temperature value being obtained at a time-point at which the first time is passed from the operation starting time-point of the air conditioning device, and setting a fourth threshold value different from the third threshold value as a threshold value based on the first temperature value and the second temperature value being obtained at a time-point at which the second time which is different from the first time is passed from the operation starting time-point of the air conditioning device.

Here, the setting a fourth threshold value may include setting the fourth threshold value to the third threshold value to a relatively small value based on the second time being relatively longer than the first time.

The control method according to an embodiment of the disclosure may further include transmitting the first temperature value to the external server, and the controlling may include controlling, based on a control signal for controlling an operation of the compressor being received from the external server according to the difference value between the first temperature value and the second temperature value, an operation of the compressor based on the control signal.

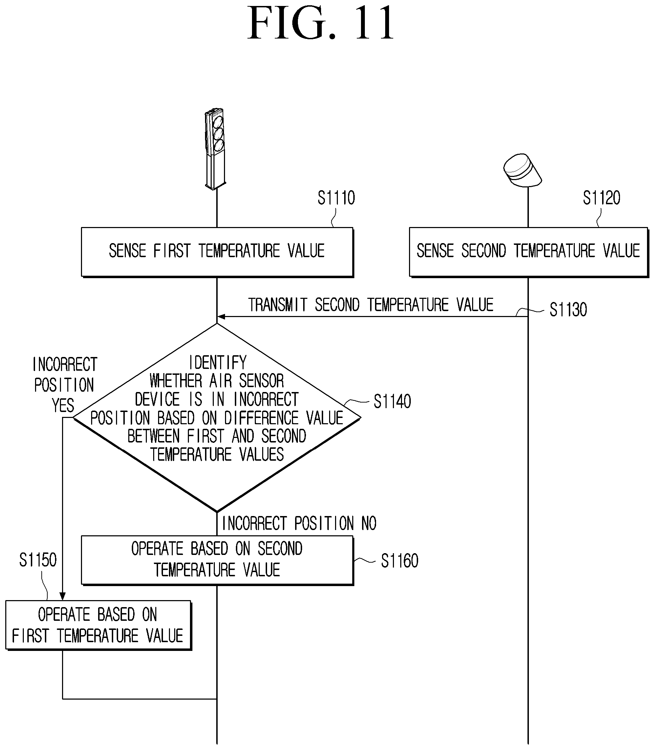

FIG. 11 is a sequence diagram illustrating communication between an air conditioning device and an air sensor device according to an embodiment of the disclosure.

Descriptions for steps that overlap with the steps described in FIG. 9 and FIG. 10 from among the steps shown in FIG. 11 will be omitted.

According to an embodiment of the disclosure, the air conditioning device 100 may sense the first temperature value at a time-point at which the first time is passed after the air conditioning device 100 has started operation (S1110).

According to an embodiment of the disclosure, the air sensor device 200 may sense the second temperature value at a time-point at which the first time is passed after the air conditioning device 100 has started operation (S1120), and transmit the sensed second temperature value to the air conditioning device 100 (S1130).

According to an embodiment of the disclosure, the air conditioning device 100 may identify whether the air sensor device 200 is in an incorrect position based on the difference value between the first temperature value and the second temperature value (S1140).

For example, the air conditioning device 100 may identify, based on the difference value between the first temperature value and the second temperature value being greater than or equal to the threshold value, the position of the air sensor device 200 as in the incorrect position (S1140: Y).

In another example, the air conditioning device 100 may identify, based on the difference value between the first temperature value and the second temperature value being less than the threshold value, the position of the air sensor device 200 as in a correct position (not the incorrect position) (S 1140: N).

Here, the incorrect position may mean that the air sensor device 200 is disposed at a position at which air discharged from the air conditioning device 100 directly reaches, and the temperature sensed by the air sensor device 200 is somewhat different from the indoor temperature at which the user is positioned, or somewhat different from the decreasing or increasing indoor temperature (or, a sensory temperature of the user) according to an operation of the air conditioning device 100.

Then, if the air conditioning device 100 identifies the position of the air sensor device 200 as in the incorrect position (S 1140: Y), the air conditioning device 100 may operate based on the first temperature value.

In another example, if the air conditioning device 100 identifies the position of the air sensor device 200 as in the correct position (S1140: N), the air conditioning device 100 may operate based on the second temperature value. Accordingly, the air conditioning device 100 may identify the second temperature value sensed by the air sensor device 200 as the surrounding temperature of the user (the sensory temperature of the user), and there may be an advantage of the air conditioning device 100 operating taking into consideration the surrounding temperature of the user rather than operating taking into consideration only the first temperature value sensed by the air conditioning device 100.

However, the various embodiments of the disclosure is not only applicable to the air conditioning device, but to electronic devices of all types.

Meanwhile, the various embodiments described above may be implemented in a recordable medium which is readable by a computer or a device similar to the computer using software, hardware, or a combination thereof. In some cases, the embodiments described herein may be implemented by the processor itself. According to a software implementation, embodiments such as the procedures and functions described herein may be implemented with separate software modules. Each of the software modules may perform one or more functions and operations described herein.

Meanwhile, computer instructions for performing processing operations in the electronic device according to the various embodiments described above may be stored in a non-transitory computer-readable medium. The computer instructions stored in this non-transitory computer-readable medium may cause a specific device to perform a processing operation of the display device 100 according to the above-described various embodiments when executed by a processor of the specific device.

The non-transitory computer readable medium may refer to a medium that stores data semi-permanently rather than storing data for a very short time, such as a register, a cache, a memory, or the like, and is readable by a device. Specific examples of the non-transitory computer readable medium may include, for example, and without limitation, a compact disc (CD), a digital versatile disc (DVD), a hard disc, a Blu-ray disc, a USB, a memory card, a ROM, and the like.

While the disclosure has been illustrated and described with reference to various embodiments thereof, it will be understood that the various embodiments are intended to be illustrative, not limiting. It will be understood by those skilled in the art that various changes in form and details may be made therein without departing from the true spirit and full scope of the disclosure, including the appended claims and their equivalents.

**Claims**

1. An air conditioning device, comprising:

a communication interface comprising circuitry;
a temperature sensor;
a compressor; and
a processor configured to control an operation of the compressor based on a setting temperature value set from the air conditioning device, a first temperature value obtained by the temperature sensor, and a second temperature value received from an air sensor device through the communication interface,
wherein the processor is configured to
identify a difference value between the first temperature value and the second temperature value, and control, based on the identified difference value being greater than or equal to a threshold value, an operation of the compressor based on the setting temperature value and the first temperature value.

2. The air conditioning device of claim 1, wherein

   the processor is configured to
   identify, based on the identified difference value being greater than or equal to a threshold value, a relationship between a position at which air discharged from the air conditioning device reaches and a current position of the air sensor device, and transmit information that guides of a position change of the air sensor device based on the identified relationship to the air sensor device through the communication interface.

3. The air conditioning device of claim 2, wherein

   the processor is configured to
   identify, based on the identified difference value being greater than or equal to a threshold value, that the air sensor device is disposed at a position at which air discharged from the air conditioning device directly reaches, and transmit information that guides of the position change of the air sensor device to the air sensor device through the communication interface.

4. The air conditioning device of claim 1, wherein

   the processor is configured to
   identify, based on the air conditioning device operating in a cooling mode, whether a difference value of subtracting the second temperature value from the first temperature value is greater than or equal to a first threshold value, and
   operate, based on the identified difference value being greater than or equal to the first threshold value, the compressor for a cooling function to be performed based on the setting temperature and the first temperature value.

5. The air conditioning device of claim 4, wherein

   the processor is configured to
   operate, based on the difference value being less than the first threshold value, the compressor for the cooling function to be performed based on the setting temperature and the second temperature value.

6. The air conditioning device of claim 1, wherein

   the processor is configured to
   identify, based on the air conditioning device operating in a heating mode, whether a difference value of subtracting the first temperature value from the second temperature value is greater than or equal to a second threshold value, and
   operate, based on the identified difference value being greater than or equal to the second threshold value, the compressor for a heating function to be performed based on the setting temperature and the first temperature value.

7. The air conditioning device of claim 6, wherein

   the processor is configured to
   operate, based on the difference value being less than the second threshold value, the compressor for the heating function to be performed based on the setting temperature and the second temperature value.

8. The air conditioning device of claim 1, wherein

   the processor is configured to
   set a third threshold value as the threshold value based on the first temperature value and the second temperature value being obtained at a time-point at which a first time is passed from an operation starting time-point of the air conditioning device, and
   set a fourth threshold value different from the third threshold value as the threshold value based on the first temperature value and the second temperature value being obtained at a time-point at which a second time different from the first time is passed from an operation starting time-point of the air conditioning device.

9. The air conditioning device of claim 8, wherein

the processor is configured to
set the fourth threshold value to the third threshold value to a relatively small value based on the second time being relatively longer than the first time.

10. The air conditioning device of claim 1, wherein

the processor is configured to
transmit the first temperature value to an external server through the communication interface, and
control, based on a control signal for controlling an operation of the compressor being received from the external server according to the difference value of the first temperature value and the second temperature value, an operation of the compressor based on the control signal.

11. A control method of an air conditioning device which comprises a temperature sensor, the method comprising:

obtaining a first temperature value through the temperature sensor; and
controlling an operation of a compressor of the air conditioning device based on a setting temperature value set from the air conditioning device, the first temperature value, and a second temperature value received from an air sensor device,
wherein the controlling comprises
identifying a difference value between the first temperature value and the second temperature value, and
controlling, based on the identified difference value being greater than or equal to a threshold value, an operation of the compressor based on the setting temperature value and the first temperature value.

12. The method of claim 11, further comprising:

identifying, based on the identified difference value being greater than or equal to the threshold value, a relationship between a position at which air discharged from the air conditioning device reaches and a current position of the air sensor device; and
transmitting information that guides of a position change of the air sensor device to the air sensor device based on the identified relationship.

13. The method of claim 12, wherein

the identifying comprises
identifying, based on the identified difference value being greater than or equal to the threshold value, that the air sensor device is disposed at a position at which air discharged from the air conditioning device directly reaches.

14. The method of claim 11, wherein

the controlling comprises
identifying, based on the air conditioning device operating in a cooling mode, whether a difference value of subtracting the second temperature value from the first temperature value is greater than or equal to a first threshold value; and
operating, based on the identified difference value being greater than or equal to the first threshold value, the compressor for a cooling function to be performed based on the setting temperature and the first temperature value.

15. The method of claim 14, wherein

the controlling comprises
operating, based on the difference value being less than the first threshold value, the compressor for the cooling function to be performed based on the setting temperature and the second temperature value.

# FIG. 1

100

200

# FIG. 2

<u>100</u>

110

COMMUNICATION
INTERFACE

120

TEMPERATURE
SENSOR

130

COMPRESSOR

140

PROCESSOR

# FIG. 3

200

210 220

| SENSOR | ←→ | PROCESSOR |

# FIG. 4

27°C
INDOOR TEMPERATURE
10

DESIRED
TEMPERATURE
22°C
20

100

# FIG. 5

< COOLING MODE >

TEMPERATURE

AIR CONDITIONING DEVICE
SENSING TEMPERATURE

AIR SENSOR DEVICE
SENSING TEMPERATURE

SETTING
TEMPERATURE

EXCESSIVE COOLING

TIME

# FIG. 6

# FIG. 7

< COOLING MODE >

27°C
INDOOR TEMPERATURE — 10

DESIRED
TEMPERATURE — 20
22°C

100

200

AIR SENSOR DEVICE
SENSING TEMPERATURE

AIR CONDITIONING DEVICE
SENSING TEMPERATURE

TEMPERATURE

SETTING
TEMPERATURE

TIME

# FIG. 8

< HEATING MODE >

100

200

AIR SENSOR DEVICE
SENSING TEMPERATURE

AIR CONDITIONING DEVICE
SENSING TEMPERATURE

TEMPERATURE

SETTING
TEMPERATURE

TIME

# FIG. 9

< COOLING MODE >

```
                    ┌─────────────┐
                    │    START    │
                    └──────┬──────┘
                           │
                           ▼            S910
                    ╱──────────────╲
                  ╱     FIRST        ╲
                ╱ TEMPERATURE VALUE-SECOND╲      N
              ╱ TEMPERATURE VALUE ≥ THRESHOLD ╲──────────────┐
                ╲         VALUE         ╱                     │
                  ╲                   ╱                       │
                    ╲──────────────╱                         │
                           │ Y                               │
                           │        S920                     │        S930
                           ▼                                 ▼
        ┌────────────────────────────────┐   ┌────────────────────────────────┐
        │ OPERATE BASED ON SETTING TEMPERATURE │ │ OPERATE BASED ON SETTING TEMPERATURE │
        │ VALUE AND FIRST TEMPERATURE VALUE │   │ VALUE AND SECOND TEMPERATURE VALUE │
        └────────────────┬───────────────┘   └────────────────┬───────────────┘
                         │◄────────────────────────────────────┘
                         ▼
                  ┌─────────────┐
                  │     END     │
                  └─────────────┘
```

# FIG. 10

< HEATING MODE >

```
        ┌──────────┐
        │  START   │
        └────┬─────┘
             │
             ▼                              S1010
          ╱─────────╲
         ╱  SECOND    ╲
        ╱ TEMPERATURE  ╲
       ╱ VALUE-FIRST    ╲        N
      ╱ TEMPERATURE VALUE ≥ THRESHOLD ──────────────┐
       ╲     VALUE      ╱                            │
        ╲             ╱                              │
         ╲───────────╱                               │
             │ Y                                     │
             │        S1020                          │              S1030
             ▼                                       ▼
┌────────────────────────────┐        ┌────────────────────────────┐
│ OPERATE BASED ON SETTING    │        │ OPERATE BASED ON SETTING    │
│ TEMPERATURE VALUE AND FIRST │        │ TEMPERATURE VALUE AND SECOND│
│ TEMPERATURE VALUE           │        │ TEMPERATURE VALUE           │
└─────────────┬──────────────┘        └──────────────┬─────────────┘
              │◄─────────────────────────────────────┘
              ▼
        ┌──────────┐
        │   END    │
        └──────────┘
```

# FIG. 11

SENSE FIRST TEMPERATURE VALUE — S1110

SENSE SECOND TEMPERATURE VALUE — S1120

TRANSMIT SECOND TEMPERATURE VALUE — S1130

IDENTIFY WHETHER AIR SENSOR DEVICE IS IN INCORRECT POSITION BASED ON DIFFERENCE VALUE BETWEEN FIRST AND SECOND TEMPERATURE VALUES — S1140

INCORRECT POSITION YES

INCORRECT POSITION NO

OPERATE BASED ON SECOND TEMPERATURE VALUE — S1160

OPERATE BASED ON FIRST TEMPERATURE VALUE — S1150

## INTERNATIONAL SEARCH REPORT

| | International application No. |
|---|---|
| | **PCT/KR2023/002423** |

**A. CLASSIFICATION OF SUBJECT MATTER**

**F24F 11/62**(2018.01)i; **F24F 11/89**(2018.01)i; **F24F 11/50**(2018.01)i; **F25B 49/02**(2006.01)i; **G01N 33/00**(2006.01)i; **F24F 110/10**(2018.01)i; **F24F 110/20**(2018.01)i; **F24F 110/30**(2018.01)i; **F24F 110/64**(2018.01)i

According to International Patent Classification (IPC) or to both national classification and IPC

**B. FIELDS SEARCHED**

Minimum documentation searched (classification system followed by classification symbols)

F24F 11/62(2018.01); F24F 11/00(2006.01); F24F 11/02(2006.01); F24F 11/49(2018.01); F25B 49/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 공기 조화 장치(air conditioner), 에어 센서 장치(air sensor device), 온도(temperature), 차이 값(difference value), 위치(position)

**C. DOCUMENTS CONSIDERED TO BE RELEVANT**

| Category* | Citation of document, with indication, where appropriate, of the relevant passages | Relevant to claim No. |
|---|---|---|
| X | KR 10-2001933 B1 (LG ELECTRONICS INC.) 02 October 2019 (2019-10-02)<br>See paragraphs [0054]-[0138] and figures 2-4. | 1,4-11,14,15 |
| Y | | 2,3,12,13 |
| Y | KR 10-1165067 B1 (ELECTRONICS AND TELECOMMUNICATIONS RESEARCH INSTITUTE) 12 July 2012 (2012-07-12)<br>See paragraph [0017]. | 2,3,12,13 |
| A | JP 5575014 B2 (MITSUBISHI ELECTRIC CORP.) 20 August 2014 (2014-08-20)<br>See paragraphs [0015]-[0045] and figures 1-7. | 1-15 |
| A | KR 10-0159233 B1 (LG ELECTRONICS INC.) 15 January 1999 (1999-01-15)<br>See claims 1-4. | 1-15 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 May 2023** | **30 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/002423**

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| A | US 2020-0018507 A1 (MITSUBISHI ELECTRIC CORPORATION) 16 January 2020 (2020-01-16) See paragraphs [0045]-[0173] and figures 1A-13. | 1-15 |

Form PCT/ISA/210 (second sheet) (July 2022)

**INTERNATIONAL SEARCH REPORT**
Information on patent family members

International application No.

**PCT/KR2023/002423**

| Patent document cited in search report | | | Publication date (day/month/year) | Patent family member(s) | | | Publication date (day/month/year) |
|---|---|---|---|---|---|---|---|
| KR | 10-2001933 | B1 | 02 October 2019 | KR 10-2018-0041274 | | A | 27 April 2018 |
| KR | 10-1165067 | B1 | 12 July 2012 | KR 10-2010-0066897 | | A | 18 June 2010 |
| JP | 5575014 | B2 | 20 August 2014 | JP 2012-184877 | | A | 27 September 2012 |
| KR | 10-0159233 | B1 | 15 January 1999 | KR 10-1997-0070813 | | A | 07 November 1997 |
| US | 2020-0018507 | A1 | 16 January 2020 | CN | 110546438 | A | 06 December 2019 |
| | | | | CN | 110546438 | B | 30 July 2021 |
| | | | | EP | 3608599 | A1 | 12 February 2020 |
| | | | | EP | 3608599 | A4 | 08 April 2020 |
| | | | | EP | 4123236 | A1 | 25 January 2023 |
| | | | | JP | 6719660 | B2 | 08 July 2020 |
| | | | | US | 11181293 | B2 | 23 November 2021 |
| | | | | WO | 2018-185937 | A1 | 11 October 2018 |

Form PCT/ISA/210 (patent family annex) (July 2022)